# EUROPÄISCHE PATENTSCHRIFT

(11) **EP 3 500 319 B1**
(45) Veröffentlichungstag und Bekanntmachung des Hinweises auf die Patenterteilung: **30.09.2020**
(21) Anmeldenummer: 17754333.7
(22) Anmeldetag: 14.08.2017
(51) Int. Cl.: A61M 1/16

(54) **VORRICHTUNG ZUR BEREITSTELLUNG VON DIALYSIERFLÜSSIGKEIT UND DIALYSEVORRICHTUNG**
DEVICE FOR PREPARING DIALYSIS FLUID AND DIALYSIS DEVICE
DISPOSITIF POUR PRÉPARER UN LIQUIDE DE DIALYSE ET DISPOSITIF DE DIALYSE

(30) Priorität: 20.08.2016 DE 102016010222
(43) Veröffentlichungstag der Anmeldung: 26.06.2019
(73) Patentinhaber: Fresenius Medical Care Deutschland GmbH, 61352 Bad Homburg v.d.H (DE)
(72) Erfinder: GLASER, Benedict, 97421 Schweinfurt (DE)
(74) Vertreter: Oppermann, Frank
(86) Internationale Anmeldenummer: PCT/EP2017/070635
(87) Internationale Veröffentlichungsnummer: WO 2018/036859

(56) Entgegenhaltungen:
- EP-A1- 1 491 222
- WO-A1-2005/118485
- WO-A2-02/32476
- DE-A1- 3 333 362
- US-A1- 2008 202 591

## Beschreibung

Die Erfindung betrifft eine Vorrichtung zur Bereitstellung von Dialysierflüssigkeit mit einer Bilanziereinrichtung, die mindestens eine Bilanzkammer zum Bilanzieren von frischer und gebrauchter Dialysierflüssigkeit aufweist, und einer Dosiereinrichtung zum Befüllen der mindestens einen Bilanzkammer mit Permeat und Konzentraten in einem vorgegebenen Mischungsverhältnis zur Herstellung von Dialysierflüssigkeit. Darüber hinaus betrifft die Erfindung eine Dialysevorrichtung mit einer Vorrichtung zur Bereitstellung von Dialysierflüssigkeit.

Zur Entfernung von harnpflichtigen Substanzen und zum Flüssigkeitsentzug werden beim chronischen Nierenversagen verschiedene Verfahren zur apparativen Blutreinigung bzw. Blutbehandlung eingesetzt. Wegen der großen Austauschmengen besteht die Notwendigkeit der exakten Bilanzierung von frischer Dialysierflüssigkeit und gebrauchter Dialysierflüssigkeit unter Berücksichtigung der über die Membran des Dialysators dem Patienten entzogenen Menge an Flüssigkeit. An die Genauigkeit der Bilanzierung werden sehr hohe Anforderungen gestellt.

Zum Stand der Technik gehören Bilanziersysteme, die über Bilanzkammern verfügen, die von einer flexiblen Membran in zwei Bilanzkammerhälften unterteilt sind. Die DE-A-28 38 414 C2 beschreibt eine Dialysevorrichtung mit einem Bilanziersystem, das über zwei Bilanzkammern verfügt, die von einer Membran jeweils in zwei Bilanzkammerhälften unterteilt sind, die in aufeinanderfolgenden Bilanzkammer-Takten aufeinanderfolgender Arbeitszyklen wechselweise betrieben werden, so dass eine Bilanzkammerhälfte mit frischer Dialysierflüssigkeit unter Verwerfung gebrauchter Dialysierflüssigkeit aus der jeweils anderen Kammerhälfte befüllt werden.

Zur Herstellung von Dialysierflüssigkeit finden im Allgemeinen vorgefertigte Dialysatkonzentrate Verwendung, die mit einer vorgegebenen Menge an Wasser verdünnt werden. Als Konzentrate werden saure Konzentrate und basische Konzentrate verwendet.

Das Wasser und die Konzentrate werden in einem sogenannten Mischkreis der Dialysevorrichtung gemischt, der das Bilanziersystem umfasst. In dem Mischkreis werden die Konzentrate dem Permeat (Reinwasser) in einem exakt vorgegebenen volumetrischen Verhältnis zudosiert, wobei sich die Volumina direkt aus den verwendeten Konzentraten und den Vorgaben für die herzustellende Dialysierflüssigkeit ableiten. Die bekannten Mischkreise verfügen für jedes Konzentrat über eine eigene Dosierpumpe, die das jeweilige Konzentrat in den einzelnen Bilanzkammer-Takten den Bilanzkammern gleichzeitig zuführen. Dabei stellt die Zuführung der Konzentrate innerhalb eines Bilanzkammer-Taktes eine technische Herausforderung dar. In der Praxis liegt die Füllzeit einer Bilanzkammer mit einem Volumen von 30ml bei einem Permeatfluss von 1400 ml/min bei nur 1,3 s.

Die EP 1 491 222 A1 beschreibt eine Vorrichtung zur extrakorporalen Blutbehandlung mit einer Einrichtung zur Bereitstellung frischer Dialysierflüssigkeit, die eine Frischwasserquelle sowie zwei Dialysierflüssigkeitskonzentratquellen aufweist. Die Wasserquelle ist über eine Wasserleitung und die Konzentratquellen sind über Konzentratleitungen und mit einem Mischpunkt verbunden, von dem die Dialysierflüssigkeitzuführleitung abgeht. In die Wasser- und Konzentratleitungen sind jeweils Proportionierungspumpen geschaltet.

Die DE 33 33 362 A1 beschreibt eine Vorrichtung zur Peritonealdialyse mit einer Einrichtung zur Bereitstellung von frischer Dialysierflüssigkeit, die einen Behälter für ein Elektrolytkonzentrat und einen Behälter für eine osmotisch wirksame Substanz aufweist. Das Elektrolytkonzentrat und die osmotisch wirksame Substanz werden jeweils mittels einer Pumpe zu einer Mischeinrichtung gefördert, die mit einem Frischwasseranschluss verbunden ist.

Aus der WO 2005/118485 A1 ist Dialysevorrichtung bekannt, die über eine Einrichtung zur Bereitstellung frischer Dialysierflüssigkeit verfügt, die auch separate Pumpen zum Fördern der Konzentrate aufweist.

Die WO 02/32476 A2 offenbart eine Dialysevorrichtung mit einer nicht näher beschriebenen Mischeinrichtung, die über Leitungen mit einer Wasserquelle und einer Konzentratquelle verbunden ist.

Der Erfindung liegt die Aufgabe zugrunde, eine Vorrichtung zur Bereitstellung von Dialysierflüssigkeit für eine Dialysevorrichtung zu schaffen, die eine genaue Dosierung von Permeat und Konzentraten erlaubt. Darüber hinaus liegt der Erfindung die Aufgabe zugrunde, eine Dialysevorrichtung bereitzustellen, bei der die Dialysierflüssigkeit in einem genauen Mischungsverhältnis aus Permeat und Konzentraten hergestellt wird.

Die Lösung dieser Aufgabe erfolgt erfindungsgemäß mit den Merkmalen des Patentanspruchs 1. Die abhängigen Ansprüche betreffen vorteilhafte Ausführungsformen der Erfindung.

Die erfindungsgemäße Vorrichtung zur Bereitstellung von Dialysierflüssigkeit und die erfindungsgemäße Dialysevorrichtung mit der Vorrichtung zur Bereitstellung von Dialysierflüssigkeit zeichnen sich durch einen vereinfachten Aufbau und geringer Herstellungskosten aus. Darüber hinaus ist der Aufwand für die Wartung der Vorrichtung geringer. Ein weiterer Vorteil liegt in der kompakten Bauweise und dem geringeren Gewicht.

Die Vorrichtung zur Bereitstellung von Dialysierflüssigkeit verfügt über eine Bilanziereinrichtung, die mindestens eine Bilanzkammer zum Bilanzieren von frischer und gebrauchter Dialysierflüssigkeit aufweist, und eine Dosiereinrichtung zum Befüllen der mindestens einen Bilanzkammer mit Permeat und Konzentraten in einem vorgegebenen Mischungverhältnis zur Herstellung von Dialysierflüssigkeit.

Die Dosiereinrichtung ist derart ausgebildet, dass vorgegebene Mengen an Konzentraten in aufeinanderfolgenden Arbeitstakten in die mindestens eine Bilanzkammer gefördert werden. Die vorgegebenen Mengen an Konzentraten werden nicht gleichzeitig zudosiert, sondern die Konzentrate werden mit nur einer einzigen Dosierpumpe in aufeinanderfolgenden Arbeitstakten gefördert. Folglich sind nicht mehrere Dosierpumpen erforderlich. Dies ist insofern von Vorteil, als die Dosierpumpe ein relatives teures Bauteil des Mischkreises darstellt. Mit nur einer Dosierpumpe entfallen auch weitere Komponenten, die für weitere Dosierpumpen erforderlich wären. In der Praxis ergibt sich ein vereinfachter, kompakter Aufbau mit geringeren Wartungskosten.

Eine bevorzugte Ausführungsform der Dosiereinrichtung sieht eine Dosierpumpe mit einem Einlass und einem Auslass und eine Verteileranordnung vor, die Einlässe zum Zuführen der Konzentrate und einen Auslass aufweist. Der Auslass der Verteileranordnung ist mit dem Einlass der Dosierpumpe verbunden ist. An den Einlässen der Verteileranordnung können Flüssigkeitsleitungen zum Ansaugen der Konzentrate aus Konzentratquellen angeschlossen sein. Die Konzentratquellen können Behältnisse sein, beispielsweise Kanister oder Beutel, in denen das Konzentrat, beispielsweise ein Säurekonzentrat und ein basisches Konzentrat, insbesondere Bikarbonat, bereitgestellt werden.

Die Dosierpumpe ist vorzugsweise eine Membranpumpe, insbesondere Exzentermembranpumpe. Die Membranpumpe erlaubt eine genaue und schnelle Dosierung innerhalb des relativ kurzen Bilanzkammertaktes. Zur Dosierung des Konzentrats können aber auch andere Pumpen mit einer ausreichend hohen Dosiergenauigkeit und einer ausreichend hohen Förderrate verwendet werden. Sollte die Förderrate der verwendeten Dosierpumpe nicht ausreichend sein, kann eine vollständige Befüllung der Bilanzkammer auch durch eine Begrenzung des maximalen Dialysierflüssigkeitsflusses sichergestellt werden.

Bei einer weiteren bevorzugten Ausführungsform weist die Verteileranordnung Absperrorgane zum Öffnen und Schließen der Einlässe auf. Die Absperrorgane werden von einer Steuereinheit angesteuert, die derart konfiguriert ist, dass die Absperrorgane nicht gleichzeitig sondern nacheinander jeweils für ein vorgegebenes Zeitintervall geöffnet werden. Die Dauer der vorgegebenen Zeitintervalle sollte kleiner als das Zeitintervall eines Bilanzkammer-Taktes sein, so dass die Zudosierung sämtlicher Konzentrate innerhalb eines Bilanzkammer-Taktes erfolgen kann.

Eine besonders bevorzugte Ausführungsform sieht vor, dass die Bilanziereinrichtung eine erste und eine zweite Bilanzkammer aufweist, die durch eine Membran jeweils in eine erste und zweite Bilanzkammerhälfte aufgeteilt sind, so dass beim Befüllen einer Kammerhälfte mit frischer Dialyisierflüssigkeit gebrauchte Dialysierflüssigkeit aus der anderen Kammerhälfte verdrängt wird oder beim Befüllen einer Kammerhälfte mit gebrauchter Dialyisierflüssigkeit frische Dialysierflüssigkeit aus der anderen Kammerhälfte verdrängt wird. Der Bilanziereinrichtung wird frische Dialysierflüssigkeit über eine Zuführleitung zugeführt und gebrauchte Dialysierflüssigkeit wird aus der Bilanziereinrichtung über eine Abführleitung abgeführt. Die Bilanziereinrichtung ist derart ausgebildet ist, dass zur Bilanzierung von frischer und gebrauchter Dialysierflüssigkeit in aufeinanderfolgenden Bilanzkammer-Takten von aufeinanderfolgenden Arbeitszyklen die Bilanzkammerhälften wechselweise befüllt bzw. entleert werden. Derartige Bilanzkammersysteme gehören zum Stand der Technik.

Die frische Dialysierflüssigkeit wird vorzugsweise dadurch hergestellt, dass an einem Mischpunkt Permeat und Konzentrat zugeführt werden. Der Mischpunkt kann vor der Bilanzkammer liegen, so dass die aus Permeat und Konzentrat hergestellte Dialysierflüssigkeit in die Bilanzkammer strömt, wo sich Permeat und Konzentrate noch weiter vermischen können.

Bei einer weiteren besonders bevorzugten Ausführungsform geht die Zuführleitung der Bilanziereinrichtung für frische Dialysierflüssigkeit von einem Mischpunkt ab, zu dem eine Permeat-Förderleitung zum Fördern von Permeat aus einer Permeatquelle und eine von dem Auslass der Dosiereinrichtung abgehende Konzentrat-Förderleitung führt.

Die Dosiereinrichtung kann einen Spülmodus für die Dosierpumpe vorsehen, die nacheinander unterschiedliche Konzentrate fördert, um zu verhindern, dass in der Dosierpumpe verbleibende Reste der Konzentrate miteinander reagieren können. Die Spülung der Dosierpumpe erfolgt vorzugsweise mit einer Spülflüssigkeit, die dem Einlass der Dosierpumpe über eine Spülleitung zugeführt wird. Zur Unterbrechung des Spülzyklus kann in der Zuführleitung ein Absperrorgan vorgesehen sein, das von der Steuereinheit angesteuert werden kann. Die Steuereinheit ist vorzugsweise derart konfiguriert, dass zwischen einzelnen Arbeitstakten, in denen eine vorgegebene Menge eines Konzentrates in die mindestens eine Bilanzkammer gefördert wird, das in der Spülleitung angeordnete Absperrorgan geöffnet und die Absperrorgane zum Öffnen bzw. Schließen der Einlässe der Verteileranordnung geschlossen sind.

Die erfindungsgemäße Dialysevorrichtung verfügt über einen extrakorporalen Blutkreislauf und ein Dialysierflüssigkeitssystem, das von dem extrakorporalen Blutkreislauf durch eine semipermeable Membran eines Filters oder Dialysators getrennt ist, der eine Blutkammer und eine Dialysierflüssigkeitskammer aufweist, wobei die Blutkammer Teil des extrakorporalen Blutkreislaufs und die Dialysierflüssigkeitskammer Teil des Dialysierflüssigkeitssystems ist. Bei der erfindungsgemäßen Dialysevorrichtung ist die Vorrichtung zur Bereitstellung von Dialysierflüssigkeit Bestandteil des Dialysierflüssigkeitssystems der Dialysevorrichtung. Daher kann die Vorrichtung zur Bereitstellung von Dialysierflüssigkeit auch von Komponenten der Dialysevorrichtung Gebrauch machen. Beispielsweise kann die Steuereinheit der Vorrichtung zur Bereitstellung von Dialysierflüssigkeit Bestandteil der zentralen Steuer- und Recheneinheit der Dialysevorrichtung sein.

Nachfolgend wird ein Ausführungsbeispiel der Erfindung unter Bezugnahme auf die Zeichnungen näher erläutert.

Es zeigen:
- Fig. 1: die wesentlichen Komponenten der erfindungsgemäßen Dialysevorrichtung mit der erfindungsgemäßen Vorrichtung zur Bereitstellung von Dialysierflüssigkeit in vereinfachter schematische Darstellung,
- Fig. 2: der Fluss der Konzentrate während eines Bilanzkammer-Taktes der Bilanziereinrichtung ohne Spülphase, und
- Fig. 3: der Fluss der Konzentrate während eines Bilanzkammer-Taktes der Bilanziereinrichtung mit Spülphase.

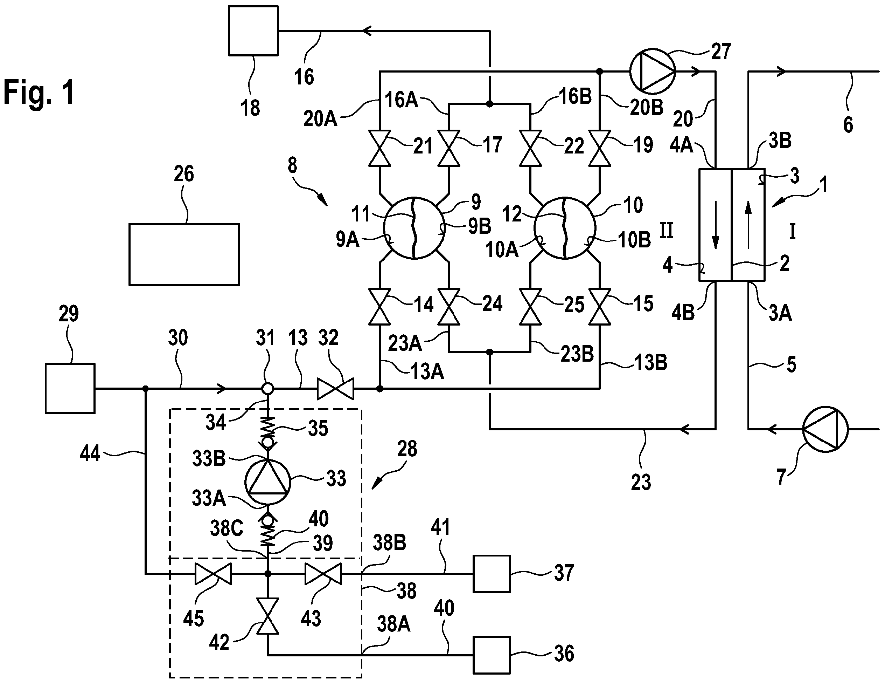

Fig. 1 zeigt in vereinfachter schematischer Darstellung eine Dialysiervorrichtung, die über eine Vorrichtung zur Bereitstellung von Dialysierflüssigkeit verfügt. In Fig. 1 sind nur die für die Erfindung wesentlichen Komponenten der Dialysiervorrichtung dargestellt.

Die Dialysevorrichtung weist einen Dialysator 1 auf, der durch eine semipermeable Membran 2 in eine Blutkammer 3 und eine Dialysierflüssigkeitskammer 4 unterteilt ist. An den Einlass 3A der Blutkammer 3 ist eine Blutzuführleitung 5 und an den Auslass 3B der Blutkammer 3 ist eine Blutabführleitung 6 angeschlossen. Das Blut des Patienten wird im extrakorporalen Blutkreislauf I mit einer Blutpumpe 7 gefördert, die in der Blutzuführleitung 5 vorgesehen ist.

Das Flüssigkeitssystem II weist eine Bilanziereinrichtung 8 auf, die in der DE 28 38 414 C2 im Einzelnen beschrieben ist.

Die Bilanziereinrichtung 8 umfasst eine erste Bilanzkammer 9 und eine zweite Bilanzkammer 10. Die Bilanzkammern 9, 10 weisen jeweils ein starres Gehäuse auf, das durch eine flexible Membran 11, 12 in eine erste und eine zweite Bilanzkammerhälfte 9A, 9B bzw. 10A, 10B unterteilt ist.

Zu einem Einlass der ersten Bilanzkammerhälfte 9A der ersten Bilanzkammer 9 führt ein erster Abschnitt 13A einer Zuführleitung 13 für frische Dialysierflüssigkeit, in der ein erstes Absperrorgan 14 angeordnet ist, und zu einem Einlass der zweiten Bilanzkammerhälfte 10B der zweiten Bilanzkammer 10 führt ein zweiter Abschnitt 13B der Zuführleitung 13 für frische Dialysierflüssigkeit, in dem ein zweites Absperrorgan 15 angeordnet ist. Von einem Auslass der zweiten Bilanzkammerhälfte 9B der ersten Bilanzkammer 9 führt ein erster Abschnitt 16A einer Abführleitung 16 für verbrauchte Dialysierflüssigkeit, in dem ein drittes Absperrrogan 17 angeordnet ist, zu einem Abfluss 18. Von einem Auslass der ersten Bilanzkammerhälfte 10A der zweiten Bilanzkammer 10 führt ein zweiter Abschnitt 16B der Abführleitung 16 für verbrauchte Dialysierflüssigkeit, in dem ein viertes Absperrorgan 19 angeordnet ist, zu dem Abfluss 18.

Ein Auslass der ersten Bilanzkammerhälfte 9A der ersten Bilanzkammer 9 und ein Auslass der zweiten Bilanzkammerhälfte 10B der zweiten Bilanzkammer 10 sind über einen ersten bzw. zweiten Abschnitt 20A, 20B einer Zuführleitung 20 für frische Dialysierflüssigkeit mit einem Einlass der Dialysierflüssigkeitskammer 4A des Dialysators 1 verbunden, wobei in den ersten bzw. zweiten Abschnitt der Zuführleitung ein fünftes bzw. sechstes Absperrorgan 21, 19 angeordnet sind. Ein Auslass der Dialysierflüssigkeitskammer 4 des Dialysators 1 ist über eine Abführleitung 23 für gebrauchte Dialysierflüssigkeit mit einem Einlass der zweiten Bilanzkammerhälfte 9B der ersten Bilanzkammer 9 und mit einem Einlass der ersten Bilanzkammerhälfte 10A der zweiten Bilanzkammer 10 verbunden. In den zugehörigen Leitungsabschnitten 23A, 23B der Abführleitung 23 sind ein siebtes und achtes Absperrorgan 24, 25 angeordnet. Die Absperrorgane können elektromagnetisch oder pneumatisch betätigbare Ventile sein.

Zum Fördern der Dialysierflüssigkeit in die Dialysierflüssigkeitskammer 4 des Dialysators 1 ist in der Zuführleitung 13 eine Dialysierflüssigkeitspumpe 27 angeordnet.

Die Absperrorgane werden von einer Steuereinheit 26 angesteuert, die über nicht dargestellte Steuerleitungen mit den Absperrorganen verbunden sind, so dass die Absperrorgane betätigt werden können. Die Steuereinheit 26 steuert auch die Blutpumpe 7 und die Dialysierflüssigkeitspumpe 27 an, um die Flussraten der Pumpen einzustellen.

Die Steuereinheit kann einen allgemeinen Prozessor, einen digitalen Signalprozessor (DSP) zur kontinuierlichen Bearbeitung digitaler Signale, einen Mikroprozessor, eine anwendungsspezifische integrierte Schaltung (ASIC), einen aus Logikelementen bestehenden integrierten Schaltkreis (FPGA) oder andere integrierte Schaltkreise (IC) oder Hardware-Komponenten aufweisen, um die einzelnen Verfahrensschritte auszuführen. Auf den Hardware-Komponenten kann zur Durchführung der Verfahrensschritte ein Datenverarbeitungsprogramm (Software) laufen. Es ist auch eine Mehrzahl oder Kombination der verschiedenen Komponenten möglich.

Die Absperrorgane bilden zwei Gruppen von Absperrorganen, wobei das erste, dritte, sechste und achte Absperrorgan 14, 17, 19, 25 eine erste Gruppe und das zweite, vierte, fünfte und siebte Absperrorgan 15, 22, 21, 24 eine zweite Gruppe bilden.

Die Steuereinheit 26 ist derart konfiguriert, dass die Absperrorgane 14, 17, 19, 25 der ersten Gruppe geöffnet sind, wenn die Absperrorgane 15, 22, 21, 24 der zweiten Gruppe geschlossen sind oder umgekehrt. Dadurch arbeiten die beiden Bilanzkammern 9, 10 abwechselnd, wobei die Bilanzkammerhälften 9A, 9B bzw. 10A, 10B in aufeinanderfolgenden Bilanzkammer-Takten wechselweise befüllt bzw. entleert werden. Die Befüllung und Entleerung der Kammern erfolgt in einzelnen Bilanzkammer-Takten eines Arbeitszyklus von aufeinanderfolgenden Arbeitszyklen.

Wenn beispielsweise die Absperrorgane 14, 17, 19, 25 der ersten Gruppe geöffnet sind, wird frische Dialysierflüssigkeit in die erste Bilanzkammerhälfte 9A der ersten Bilanzkammer 9 gefördert, während gebrauchte Dialysierflüssigkeit aus der zweiten Bilanzkammerhälfte 9B der ersten Bilanzkammer 9 in den Ablauf 18 abfließt. Aus der zweiten Bilanzkammerhälfte 10B der zweiten Bilanzkammer 10 fließt frische Dialysierflüssigkeit in die Dialysierflüssigkeitskammer 4 des Dialysators 1 und gebrauchte Dialysierflüssigkeit fließt in die erste Bilanzkammerhälfte 10A der zweiten Bilanzkammer 10.

Die erfindungsgemäße Vorrichtung zur Bereitstellung von Dialysierflüssigkeit für den Dialysator der Dialysevorrichtung umfasst die Bilanziereinrichtung 8 und eine Dosiereinrichtung 28 zum Befüllen der Bilanzkammern 9, 10 der Bilanziereinrichtung 8 mit Permeat und Konzentraten in einem vorgegebenen Mischungsverhältnis.

Das Permeat (Wasser) wird in einer Permeatquelle 29 bereitgestellt. Von der Permeatquelle 29 führt eine Permeat-Förderleitung 30 zu einem Mischpunkt 31, von dem die Zuführleitung 13 für frische Dialysierflüssigkeit der Bilanziereinrichtung 8 abgeht und zu den Bilanzkammern 9, 10 führt, so dass Permeat in die Bilanzkammern der Bilanziereinrichtung strömen kann. In der Zuführleitung ist ein Absperrorgan 32 angeordnet, so dass die Zufuhr von Dialysierflüssigkeit unterbrochen werden kann.

Die Dosiereinrichtung 28 verfügt über eine Dosierpumpe 33, insbesondere eine Membranpumpe, die einen Einlass 33A (Saugseite) und einen Auslass 33B (Druckseite) aufweist. Der Auslass 33B der Membranpumpe ist über eine Konzentrat-Förderleitung 34, in der ein Rückschlagventil 35 angeordnet sein kann, mit dem Mischpunkt 31 verbunden. Bei dem vorliegenden Ausführungsbeispiel wird mit der Dosierpumpe 33 dem Permeat an dem Mischpunkt 31 eine vorgegebene Menge an einem ersten Konzentrat, beispielsweise einem Säurekonzentrat, und eine vorgegebene Menge an einem zweiten Konzentrat, beispielsweise einem basischen Konzentrat, insbesondere Bikarbonat, zudosiert. Das erste und zweite Konzentrat werden in Behältnissen 36, 37 bereitgestellt.

Darüber hinaus weist die Dosiereinrichtung 28 eine Verteileranordnung 38 auf, die einen ersten Einlass 38A zum Zuführen des ersten Konzentrats und einen zweiten Einlass 38B zum Zuführen des zweiten Konzentrats sowie einen Auslass 38C aufweist. Der Auslass 38C der Verteileranordnung 38 ist über eine Flüssigkeitsleitung 39, in der ein Rückschlagventil 40 angeordnet sein kann, mit dem Einlass 33A der Dosierpumpe 33 verbunden. An dem ersten Einlass 38A ist eine erste Ansaugleitung 40 angeschlossen, die zu dem ersten Behältnis 36 führt, und an dem zweiten Einlass 38B ist eine zweite Ansaugleitung 41 angeschlossen, die zu dem zweiten Behältnis 37 führt. Zum Öffnen bzw. Verschließen des ersten und zweiten Einlasses ist jeweils ein Absperrorgan 42, 43, beispielsweise ein elektromagnetisch betätigbares Ventil, vorgesehen.

Das Dialysierflüssigkeitssystem kann auch einen Entgasungskreislauf umfassen, der aber in Fig. 1 nicht dargestellt ist.

Bei dem vorliegenden Ausführungsbeispiel ist der Leitungsabschnitt der Permeat-Förderleitung 30 stromauf des Mischpunktes 31 über eine Spülleitung 44, in der weiteres Absperrorgan 45 angeordnet ist, mit dem Einlass 33A (Saugseite) der Dosierpumpe 33 verbunden.

Die Steuereinheit 26 der Bilanziereinrichtung 8 steuert auch die Absperrorgane 42, 43 der Verteileranordnung 28 sowie die Absperrorgane 32, 45 in der Zuführleitung 13 und der Spülleitung 44 an. Die Steuereinheit 26 ist derart konfiguriert, dass in einem Bilanzkammer-Takt während der Füllphase an dem Mischpunkt 31 die beiden Konzentrate nacheinander zudosiert werden.

Wenn beispielsweise in einem Bilanzkammer-Takt während der Füllphase die Absperrorgane 14, 17, 19, 25 der ersten Gruppe geöffnet sind, um Dialysierflüssigkeit in die erste Bilanzkammerhälfte 9A der ersten Bilanzkammer 9 zu fördern, wird das erste Absperrorgan 42 der Verteileranordnung 38 für ein vorgegebenes erstes Zeitintervall geöffnet, wobei die Dosierpumpe 33 in dem vorgegebenen ersten Zeitintervall aus dem ersten Konzentratbehälter 36 das erste Konzentrat ansaugt und an dem Mischpunkt 31 zudosiert. Wenn das erste Absperrorgan 42 wieder geschlossen ist, wird das zweite Absperrorgan 43 der Verteileranordnung 38 für ein vorgegebenes zweites Zeitintervall geöffnet, wobei die Dosierpumpe 33 in dem vorgegebenen zweiten Zeitintervall aus dem zweiten Konzentratbehälter 37 das zweite Konzentrat ansaugt und an dem Mischpunkt zudosiert. Das erste und zweite Zeitintervall dürfen nicht länger als die Dauer des Bilanzkammer-Taktes während der Füllphase sein, so dass die Konzentrate während der Füllphase zudosiert werden können. Die vollständige Befüllung der jeweiligen Bilanzkammer kann beispielsweise durch eine Überwachung des Fülldrucks sichergestellt werden. Die Dauer des ersten und zweiten Zeitintervalls bestimmt das volumetrische Verhältnis von Permeat und Konzentrat und wird von der Steuereinheit 26 in Abhängigkeit von der gewünschten Zusammensetzung der Dialysierflüssigkeit vorgegeben. Die Dosierpumpe 33 wird also in einem Bilanzkammer-Takt der Bilanziereinrichtung in aufeinanderfolgenden Arbeitstakten betrieben.

Um zu verhindern, dass Konzentratreste des ersten Konzentrats mit Konzentratresten des zweiten Konzentrats in der Dosierpumpe 33 reagieren, kann die Dosierpumpe zwischen dem ersten und zweiten Arbeitstakt, in dem jeweils ein Konzentrat zudosiert wird, gespült werden. Während der Spülphase ist das Absperrorgan 32 in der Zuführleitung 13 geschlossen und das Absperrorgan 45 in der Spüllleitung 44 offen und das erste und zweite Absperrorgan 42, 43 der Verteileranordnung 38 sind geschlossen, wobei die Dosierpumpe 33 betrieben wird, so dass Permeat in einem Spülkreislauf zirkuliert.

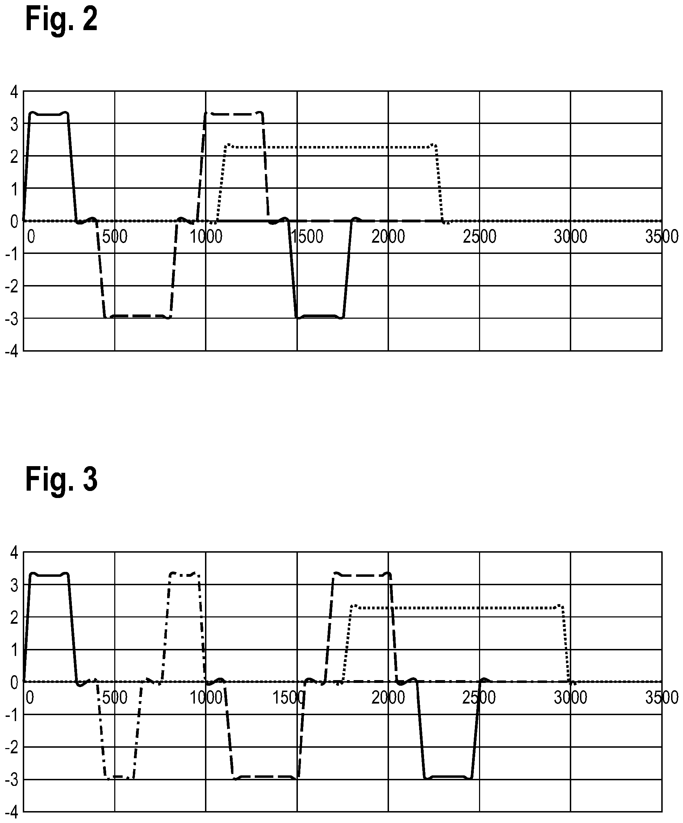

Fig. 2 zeigt den Fluss des ersten und zweiten Konzentrats während eines Bilanzkammer-Taktes der Bilanziereinrichtung 8 zum Füllen einer Bilanzkammer ohne Spülphase. Die Bilanzkammer hat bei dem vorliegenden Ausführungsbeispiel ein Volumen von 30 ml. Das Dosiervolumen des ersten Konzentrats ist bei dem Ausführungsbeispiels 828 µL und des zweiten Konzentrats 1159 µL. In Fig. 2 ist auf der Abzisse die Zeit in Millisekunden (ms) und auf der Ordinate ist der Fluss der Dosierpumpe [µL/s] / Füllfluss der Bilanzkammer [ml/min] / 10 aufgetragen. Ein negativer Fluss bedeutet, dass die Dosierpumpe 33 Konzentrat ansaugt. Der Konzentratfluss des ersten Konzentrats ist mit einer durchgezogenen Linie und der Konzentratfluss des zweiten Konzentrats ist mit einer gestrichelten Linie dargestellt. Mit einer gepunkteten Linie ist das Auffüllen der Bilanzkammer mit Permeat (Reinwasser) aus einem Entgasungskreis dargestellt. Es zeigt sich, dass die Konzentrate der Reihe nach innerhalb der Dauer des Bilanzkammer-Taktes zudosiert werden.

Fig. 3 zeigt den Fluss des ersten und zweiten Konzentrats während eines Bilanzkammer-Taktes der Bilanziereinrichtung 8 zum Füllen einer Bilanzkammer mit einer Spülphase. In Fig. 3 ist der Fluss der Spülflüssigkeit (Permeat) mit einer strichpunktierten Linie dargestellt. Die Spülung erfolgt in einem Zeitintervall zwischen der Zudosierung des ersten Konzentrats und der Zudosierung des zweiten Konzentrats. Das Spülvolumen beträgt bei dem Ausführungsbeispiel 580 µL. Aufgrund der Spülung verlängert sich die serielle Konzentratzugabe. Es zeigt sich aber, dass die Konzentratzugabe innerhalb eines Bilanzkammer-Takes abgeschlossen ist.

## Patentansprüche

1. Vorrichtung zur Bereitstellung von Dialysierflüssigkeit für eine Dialysevorrichtung mit
einer Bilanziereinrichtung (8), die mindestens eine Bilanzkammer (9, 10) zum Bilanzieren von frischer und gebrauchter Dialysierflüssigkeit aufweist, und
einer Dosiereinrichtung (28) zum Befüllen der mindestens einen Bilanzkammer (9, 10) mit Permeat und Konzentraten in einem vorgegebenen Mischungsverhältnis zur Herstellung von Dialysierflüssigkeit,
**dadurch gekennzeichnet,**
**dass** die Dosiereinrichtung (28) nur eine Dosierpumpe (33) aufweist, wobei die Dosiereinrichtung (28) derart ausgebildet ist, dass vorgegebene Mengen der Konzentrate mit der einzigen Dosierpumpe (33) in aufeinanderfolgenden Arbeitstakten in die mindestens eine Bilanzkammer (9, 10) gefördert werden.

2. Vorrichtung nach Anspruch 1, **dadurch gekennzeichnet, dass** die Dosiereinrichtung eine Dosierpumpe (33) mit einem Einlass (33A) und einem Auslass (33B) und eine Verteileranordnung (38) aufweist, die Einlässe (38A, 38B) zum Zuführen der Konzentrate aus Konzentratquellen (36, 37) und einen Auslass (38C) aufweist, wobei der Auslass (38C) der Verteileranordnung (38) mit dem Einlass (33A) der Dosierpumpe (33) verbunden ist.

3. Vorrichtung nach Anspruch 2, **dadurch gekennzeichnet, dass** die Verteileranordnung (38) Absperrorgane (42, 43) zum Öffnen und Schließen der Einlässe (38A, 38B) aufweist, wobei die Dosiereinrichtung (38) eine Steuereinheit (26) für die Absperrorgane aufweist, die derart konfiguriert ist, dass die Absperrorgane (42, 43) nacheinander jeweils für ein vorgegebenes Zeitintervall geöffnet sind.

4. Vorrichtung nach Anspruch 2 oder 3, **dadurch gekennzeichnet, dass** die Dosierpumpe (33) eine Membranpumpe ist.

5. Vorrichtung nach Anspruch 3 oder 4, **dadurch gekennzeichnet, dass** die Bilanziereinrichtung (8) aufweist:
eine erste und eine zweite Bilanzkammer (9, 10), die durch eine Membran (11, 12) jeweils in eine erste und zweite Bilanzkammerhälfte (9A, 9B; 10A, 10B) aufgeteilt sind, so dass beim Befüllen einer Kammerhälfte mit frischer Dialyisierflüssigkeit gebrauchte Dialysierflüssigkeit aus der anderen Kammerhälfte verdrängt wird oder beim Befüllen einer Kammerhälfte mit gebrauchter Dialyisierflüssigkeit frische Dialysierflüssigkeit aus der anderen Kammerhälfte verdrängt wird, und
eine Zuführleitung (13) für frische Dialysierflüssigkeit in die Bilanziereinrichtung und eine Abführleitung (16) für gebrauchte Dialysierflüssigkeit aus der Bilanziereinrichtung (8),
wobei die Bilanziereinrichtung (8) derart ausgebildet ist, dass zur Bilanzierung von frischer und gebrauchter Dialysierflüssigkeit in aufeinanderfolgenden Bilanzkammer-Takten von aufeinanderfolgenden Arbeitszyklen die Bilanzkammerhälften wechselweise befüllt bzw. entleert werden.

6. Vorrichtung nach Anspruch 5, **dadurch gekennzeichnet, dass** die Steuereinheit (26) derart konfiguriert ist, dass die Absperrorgane (42, 43) in einem Bilanzkammer-Takt nacheinander jeweils für ein vorgegebenes Zeitintervall geöffnet sind.

7. Vorrichtung nach Anspruch 5 oder 6, **dadurch gekennzeichnet, dass** die Zuführleitung (13) für frische Dialysierflüssigkeit von einem Mischpunkt (31) abgeht, zu dem eine Permeat-Förderleitung (30) zum Fördern von Permeat aus einer Permeatquelle (29) und eine von dem Auslass (33B) der Dosierpumpe (33) abgehende Konzentrat-Förderleitung (34) führt.

8. Vorrichtung nach einem der Ansprüche 3 bis 7, **dadurch gekennzeichnet, dass** zu dem Einlass (33A) der Dosierpumpe (33) eine Spülleitung (44) zum Zuführen einer Spülflüssigkeit führt, in der ein Absperrorgan (45) angeordnet ist.

9. Vorrichtung nach Anspruch 8, **dadurch gekennzeichnet, dass** die Steuereinheit (26) derart konfiguriert ist, dass zwischen einzelnen Arbeitstakten, in denen ein Absperrorgan (42, 43) für ein vorgegebenes Zeitintervall geöffnet ist, das in der Spülleitung (44) angeordnete Absperrorgan (45) geöffnet und die Absperrorgane (42, 43) zum Öffnen und Schließen der Einlässe (38A, 38B) der Verteileranordnung (38) geschlossen sind.

10. Dialysevorrichtung mit einem extrakorporalen Blutkreislauf (I) und einem Dialysierflüssigkeitssystem (II), das von dem extrakorporalen Blutkreislauf durch eine semipermeable Membran (2) eines Dialysators (1) getrennt ist, der eine Blutkammer (3) und eine Dialysierflüssigkeitskammer (4) aufweist, wobei die Blutkammer Teil des extrakorporalen Blutkreislaufs (I) und die Dialysierflüssigkeitskammer Teil des Dialysierflüssigkeitssystems (II) ist und das Dialysierflüssigkeitssystem (II) eine Vorrichtung zur Bereitstellung von Dialysierflüssigkeit nach einen der Ansprüche 1 bis 9 aufweist.

## Claims

1. Apparatus for supplying dialysate for a dialysis apparatus, comprising
a balancing device (8) which comprises at least one balancing chamber (9, 10) for balancing fresh and used dialysate, and
a metering device (28) for filling the at least one balancing chamber (9, 10) with permeate and concentrates in a specified mixing ratio for producing dialysate,
**characterised in that**
the metering device (28) comprises only a single metering pump (13), the metering device (28) being designed such that specified volumes of the concentrates are conveyed into the at least one balancing chamber (9, 10) in successive working cycles using the single metering device (33).

2. Apparatus according to claim 1, **characterised in that** the metering device comprises a metering pump (33), which has an inlet (33A) and an outlet (33B), and a distributor assembly (38) which comprises inlets (38A, 38B) for supplying the concentrates from concentrate sources (36, 37) and an outlet (38C), the outlet (38C) of the distributor assembly (38) being connected to the inlet (33A) of the metering pump (33).

3. Apparatus according to claim 2, **characterised in that** the distributor assembly (38) comprises valves (42, 43) for opening and closing the inlets (38A, 38B), the metering device (38) comprising a control unit (26) for the valves that is configured such that the valves (42, 43) are opened one after the other for a specified time period in each case.

4. Apparatus according to either claim 2 or claim 3, **characterised in that** the metering pump (33) is a membrane pump.

5. Apparatus according to either claim 3 or claim 4, **characterised in that** the balancing device (8) comprises:
a first and a second balancing chamber (9, 10), each of which is divided into a first and a second balancing chamber half (9A, 9B; 10A, 10B) by means of a membrane (11, 12), and therefore, when one chamber half is filled with fresh dialysate, used dialysate is moved out of the other chamber half, or, when one chamber half is filled with used dialysate, fresh dialysate is moved out of the other chamber half, and
a line (13) for supplying fresh dialysate to the balancing device and a line (16) for removing used dialysate from the balancing device (8),
the balancing device (8) being designed such that, in order to balance fresh and used dialysate, the balancing chamber halves are alternately filled and emptied in successive balancing chamber cycles of successive working cycles.

6. Apparatus according to claim 5, **characterised in that** the control unit (26) is configured such that the valves (42, 43) are opened one after the other in a balancing chamber cycle for a specified time period in each case.

7. Apparatus according to either claim 5 or claim 6, **characterised in that** the supply line (13) for fresh dialysate leads away from a mixing point (31), to which run both a permeate conveying line (30) for conveying permeate from a permeate source (29), and a concentrate conveying line (34) which leads away from the outlet (33B) of the metering pump (33).

8. Apparatus according to any of claims 3 to 7, **characterised in that** a rinsing line (44) for supplying a rinsing fluid runs to the inlet (33A) of the metering pump (33), in which rinsing line a valve (45) is arranged.

9. Apparatus according to claim 8, **characterised in that** the control unit (26) is configured such that, between individual working cycles, in which a valve (42, 43) is open for a specified time period, the valve (45) arranged in the rinsing line (44) is open and the valves (42, 43) for opening and closing the inlets (38A, 38B) of the distributor assembly (38) are closed.

10. Dialysis apparatus comprising an extracorporeal blood circuit (I) and a dialysate system (II) which is separated from the extracorporeal blood circuit by means of a semi-permeable membrane (2) of a dialyser (1), which dialyser comprises a blood chamber (3) and a dialysate chamber (4), wherein the blood chamber is part of the extracorporeal blood circuit (I) and the dialysate chamber is part of the dialysate system (II), and the dialysate system (II) comprises an apparatus for supplying dialysate according to any of claims 1 to 9.

## Revendications

1. Dispositif de préparation de liquide de dialyse pour un dispositif de dialyse avec un dispositif d'équilibrage (8) qui présente au moins une chambre d'équilibrage (9, 10) pour l'équilibrage de liquide de dialyse frais et usagé, et
un dispositif de dosage (28) pour le remplissage de l'au moins une chambre d'équilibrage (9, 10) avec du perméat et des concentrats dans un rapport de mélange prescrit pour la fabrication de liquide de dialyse,
**caractérisé en ce**
**que** le dispositif de dosage (28) présente seulement une pompe de dosage (33), dans lequel le dispositif de dosage (28) est réalisé de telle manière que des quantités prescrites des concentrats soient transportées avec la pompe de dosage unique (33) dans des cycles de travail successifs dans l'au moins une chambre d'équilibrage (9, 10).

2. Dispositif selon la revendication 1, **caractérisé en ce que** le dispositif de dosage présente une pompe de dosage (33) avec une entrée (33A) et une sortie (33B) et un agencement de distribution (38), qui présente des entrées (38A, 38B) pour l'amenée des concentrats de sources de concentrat (36, 37) et une sortie (38C), dans lequel la sortie (38C) de l'agencement de distribution (38) est reliée à l'entrée (33A) de la pompe de dosage (33).

3. Dispositif selon la revendication 2, **caractérisé en ce que** l'agencement de distribution (38) présente des organes de blocage (42, 43) pour l'ouverture et la fermeture des entrées (38A, 38B), dans lequel le dispositif de dosage (38) présente une unité de commande (26) pour les organes de blocage, qui est configurée de telle manière que les organes de blocage (42, 43) soient ouverts l'un après l'autre respectivement pendant un intervalle de temps prescrit.

4. Dispositif selon la revendication 2 ou 3, **caractérisé en ce que** la pompe de dosage (33) est une pompe à membrane.

5. Dispositif selon la revendication 3 ou 4, **caractérisé en ce que** le dispositif d'équilibrage (8) présente :
une première et une seconde chambre d'équilibrage (9, 10) qui sont divisées par une membrane (11, 12) respectivement en une première et seconde moitié de chambre d'équilibrage (9A, 9B ; 10A, 10B) de sorte que lors du remplissage d'une moitié de chambre avec du liquide de dialyse frais, du liquide de dialyse usagé soit déplacé de l'autre moitié de chambre ou lors du remplissage d'une moitié de chambre avec un liquide de dialyse usagé, du liquide de dialyse frais soit déplacé de l'autre moitié de chambre, et
une conduite d'amenée (13) pour du liquide de dialyse frais dans le dispositif d'équilibrage et une conduite d'évacuation (16) pour du liquide de dialyse usagé hors du dispositif d'équilibrage (8),
dans lequel le dispositif d'équilibrage (8) est réalisé de telle manière que pour l'équilibrage de liquide de dialyse frais et usagé dans des cycles de chambre d'équilibrage successifs de cycles de travail successifs les moitiés de chambre d'équilibrage soient remplies ou vidées alternativement.

6. Dispositif selon la revendication 5, **caractérisé en ce que** l'unité de commande (26) est configurée de telle manière que les organes de blocage (42, 43) soient ouverts dans un cycle de chambre d'équilibrage l'un après l'autre respectivement pendant un intervalle de temps prescrit.

7. Dispositif selon la revendication 5 ou 6, **caractérisé en ce que** la conduite d'amenée (13) pour du liquide de dialyse frais sort d'un point de mélange (31), auquel mènent une conduite de transport de perméat (30) pour le transport de perméat d'une source de perméat (29) et une conduite de transport de concentrat (34) sortant de la sortie (33B) de la pompe de dosage (33).

8. Dispositif selon l'une des revendications 3 à 7, **caractérisé en ce qu'**une conduite de rinçage (44) mène à l'entrée (33A) de la pompe de dosage (33) pour l'amenée d'un liquide de rinçage, dans laquelle un organe de blocage (45) est agencé.

9. Dispositif selon la revendication 8, **caractérisé en ce que** l'unité de commande (26) est configurée de telle manière qu'entre des cycles de travail individuels dans lesquels un organe de blocage (42, 43) est ouvert pendant un intervalle de temps prescrit, l'organe de blocage (45) agencé dans la conduite de rinçage (44) soit ouvert et les organes de blocage (42, 43) soient fermés pour l'ouverture et la fermeture des entrées (38A, 38B) de l'agencement de distribution (38).

10. Dispositif de dialyse avec un circuit sanguin extracorporel (I) et un système de liquide de dialyse (II), qui est séparé du circuit sanguin extracorporel par une membrane semi-perméable (2) d'un dialyseur (1) qui présente une chambre de sang (3) et une chambre de liquide de dialyse (4), dans lequel la chambre de sang fait partie du circuit sanguin extracorporel (I) et la chambre de liquide de dialyse fait partie du système de liquide de dialyse (II) et le système de liquide de dialyse (II) présente un dispositif de préparation de liquide de dialyse selon l'une des revendications 1 à 9.
